# EUROPEAN PATENT SPECIFICATION

(11) **EP 1 915 153 B1**
(45) Date of publication and mention of the grant of the patent: **10.04.2013**
(21) Application number: 06777710.2
(22) Date of filing: 11.07.2006
(51) Int. Cl.: A61K 31/4745, A61K 9/48, A61K 9/10, A61K 9/107, A61K 9/16, A61K 9/20, A61K 47/14, A61K 47/26, A61K 47/44

(54) **IMMEDIATE RELEASE THERAPEUTIC SYSTEMS FOR IMPROVED ORAL ABSORPTION OF 7-[(E)-TERT-BUTYLOXYIMINOMETHYL]CAMTOTHECIN**
THERAPEUTISCHE SYSTEME MIT SOFORTIGER FREISETZUNG FÜR VERBESSERTE ORALE ABSORPTION VON 7-[(E)-TERT-BUTYLOXYIMINOMETHYL] CAMPTOTHECIN
SYSTEMES THERAPEUTIQUES A LIBERATION IMMEDIATE POUR UNE MEILLEURE ABSORPTION ORALE DE LA 7-[(E)-TERT-BUTYLOXYMINOMETHYLE]CAMPTOTHECINE

(30) Priority: 04.08.2005 IT RM20050418
(43) Date of publication of application: 30.04.2008
(73) Proprietor: SIGMA-TAU Industrie Farmaceutiche Riunite S.p.A., 00144 Roma (IT)
(72) Inventor: LONGO, Antonio, I-00128 Rome (IT); PACE, Silvia, I-00144 Rome (IT); PEDRANI, Massimo, I-28 836 Gignese (VB) (IT)
(74) Representative: Spadaro, Marco
(86) International application number: PCT/EP2006/064111
(87) International publication number: WO 2007/017331

(56) References cited:
- WO-A-00/53607
- WO-A-99/06024
- WO-A-99/06031
- WO-A-2005/092302
- WO-A-2006/067092
- US-A- 5 859 023
- US-A1- 2003 065 024
- CESARE DE M ET AL: "Potent Antitumor Activity and Improved Pharmacological Profile of ST1481, a Novel 7-substituted Camptothecin" CANCER RESEARCH, AMERICAN ASSOCIATION FOR CANCER RESEARCH, BALTIMORE, MD, US, vol. 61, 2001, pages 7189-7195, XP002340217 ISSN: 0008-5472
- PETRANGOLINI G ET AL: "ANTIANGIOGENIC EFFECTS OF THE NOVEL CAMPTOTHECIN ST1481 (GIMATECAN) IN HUMAN TUMOR XENOGRAFTS" MOLECULAR CANCER RESEARCH, XX, XX, vol. 1, no. 12, October 2003 (2003-10), pages 863-870, XP008059627 ISSN: 1541-7786

## Description

### FIELD OF THE INVENTION

The present invention relates to a pharmaceutical formulation containing a camptothecin as the active principle.

### BACKGROUND OF THE INVENTION

Camptothecin is an alkaloid isolated by Wall et al (J. Am. Chem. Soc. 88, 3888-3890 (1966)) for the first time from the tree Camptotheca acuminata, a plant originating in China, of the family Nyssaceae.

The molecule consists of a pentacyclic structure with a lactone in the E ring, essential for cytotoxicity.

The broad spectrum of the antitumoral activity exhibited by the drug especially towards tumours of the colon, other solid tumours and leukaemias, led to the first clinical trials in the early 1970s. To prepare for the clinical tests on camptothecin (hereinafter referred to as CPT) which is not readily soluble in water, the National Cancer Institute (NCI) formulated the sodium salt of the compound, soluble in water (NSC100880). The clinical trial phases I and II were not completed, however, due to the excessive toxicity exhibited (haemorrhagic cystitis, gastrointestinal toxicity such as nausea, vomiting, diarrhoea and myelosuppression, especially leukopenia and thrombocytopenia).

Subsequently many analogues of CPT were synthesised with the aim of identifying compounds with less toxicity and greater solubility in water. Two drugs are on the market, irinotecan (CPT - 11), marketed as Camptosar by UpJohn (now Pfizer) and topotecan, marketed as Hymcamptamin or Thycantin, by Smith Kline & Beecham (now GSK). Other analogues exist at various stage of clinical development in phase II, such as NSC-603071 (9-aminocamptothecin), 9-NC 9-nitrocamptothecin, oral prodrug converted into 9-aminocamptothecin, GG-211 (GI 147211), and DX-8591f, the latter drugs being soluble in water. All the derivatives identified so far contain the parent structure with 5 rings, essential for cytotoxicity. It has been demonstrated that modifications in the first ring, as in the case of the drugs referred to above, increase solubility in water and mean that the drug is better tolerated.

Patent application WO97/31003 describes derivatives of camptothecins replaced in positions 7, 9 and 10. Position 7 provides for the following replacements: -CN, -CH(CN)-R₄, -CH=C(CN)-R₄, -CH₂-CH=C(CN)-R₄, -C(=NOH)-NH₂, -CH=C(NO₂)-R₄, -CH(CN)-R₅, -CH(CH₂NO₂)-R₅, 5-tetrazolyl, 2-(4,5-dihydroxazolyl), 1,2,4-oxadiazolidine-3-yl-5-one, where R₄ is hydrogenous, linear or ramified alkyl with 1 to 6 carbon atoms, nitrile, carboxyalkosyl. Of these possible compounds, WO97/31003 effectively describes the derivatives of camptothecin carried on position 7, the -CN groups and -CH=C(CN)₂, with positions 9 and 10 not replaced.

Of these, the best compound proved to be 7-nitrile (R4 = -CN), hereinafter referred to as CPT 83, with cytotoxicity on large cell pulmonary carcinoma (not SCLC, H-460). This tumour line is intrinsically resistant to cytotoxic therapy and responds only moderately to the inhibitors of topoisomerase I, irrespective of the over-expression of the target enzyme. CPT 83 is more active than topotecan, taken as a reference compound and overall offers a better pharmacological profile, also in terms of tolerability, hence has a better therapeutic index.

In patent application EP1044977 there is a description of derivatives of camptothecin which have an alkyloxyme O-replaced in position 7 and which have antitumoral activity superior to that of the reference compound topotecan.

Furthermore these derivatives of camptothecin which have an immine group in position 7, have also exhibited an improved therapeutic index. Of these compounds one of the preferred substances is 7-t-butoxyminomethylcamptothecin (CPT 184). When this substance is prepared as described in patent application No. EP1044977, from a mixture of solvents containing ethanol and pyridine a mixture of the two isomers E and Z is obtained in a ratio of 8:2.

In European patent application No. 040302465 filed in the name of the Applicant on 21 December 2004, there is a description of a stereoselective process for the preparation of 7-[(E)-t-butyloxyminomethyl]-camptothecin (also known as gimatecan). According to this process the E isomer is always obtained in a ratio of at least 95:5 compared with the Z isomer.

Furthermore it is also shown in this same patent application that this product can exist in amorphous form and in different crystalline forms and that these forms can be obtained using the same stereoselective process with the addition of further final phases of dissolution and re-precipitation using different mixtures of solvents.

These different crystalline forms were indicated as form I, form II and form III. Success in developing a drug very often also depends on the ability to find a stable formulation of the substance which allows it to be administered orally or parenterally at effective doses in the treatment desired. This ability is often limited by the intrinsic characteristics of the substance, such as only slight solubility in water.

For example in the case of the derivatives of camptothecin, almost all the derivatives which retain the lactone E ring intact are not at all readily soluble in water.

It would therefore be very useful to be able to have immediate-release pharmaceutical compounds containing 7-[(E)-t-butyloxyminomethyl]camptothecin (gimatecan) as the active principle.

This active principle, which is known to have limited solubility in biological fluids and limited absorption via the oral route, could be suitably formulated to increase bioavailability in vitro and in vivo. The active principle in question also has problems of highly variable absorption in the gastrointestinal tract.

Obtaining a preparation which is immediately available and rapidly absorbed could in principle be achieved via various well-known techniques, such as the following:
1) the use of complexes and compounds based on cyclodextrins or other polymers, in which the active principle was loaded using techniques involving dissolution in water or other organic solvents, dry comminution or in organic solvents and/or lyophilisation;
2) the use of micronisation and amorphisation processes of the active principle;
3) the use of emulsions, microemulsions (A/O, O/A), multiple emulsions (A/O/A);
4) the use of salification processes, even extemporaneous ones, or of solubilisation of the active principle itself and/or in traditional liquid formulations such as syrups, drops, solutions, soft gelatin capsules, effervescent forms;
5) the use of organic solvents and/or co-solvents (such as dioxane, dimethylacetamide, dimethylsulphoxide, dimethylisosorbide or binary or multiple systems consisting of monoethylic ether of diethylene glycol with polyethylene glycols with the addition of non-ionic tensioactive substances.

The compounds or complexes with dextrins and other polymers are expensive processes, often difficult to implement and do not guarantee the total complexation of the active principle; furthermore the ratio between the active principle and the polymer is very often a factor limiting the preparation of a pharmaceutical form which can be easily administered.

The micronisation processes often do not guarantee significant increases in plasma levels, in return increasing the apparent volumes of the powders making the processes producing the capsules, tablets and granules very difficult.

While improving the bioavailability of the drugs, the amorphisation processes produce re-crystallisation effects over time and often lead to less stability of the active principle producing negative effects on the quality of the drug.

Simple or multiple emulsions and/or microemulsions are often unstable and are unable to transport pharmacologically active quantities of the drug.

The formulative salification and/or solubilisation processes in traditional pharmaceutical forms are often unable to solubilise and/or improve the bioavailability of drugs which are not readily permeable and absorbable, as well as lyophilised products, because of re-precipitation processes of the active principle in biological fluids, thus cancelling out the advantage of a technological process capable of solubilising the drug in the pharmaceutical form. As it is necessary to produce preparations which are readily released and with potentially improved bioavailability, it becomes important to configure the preparation of a therapeutic system which guarantees the standardisation of the pharmaceutical physical state of the active principle, for rapid release of the pharmaceutical form and to reduce any deviation in the linearity of the transfer.

### DESCRIPTION OF THE INVENTION

This objective has been met in accordance with the present invention, by formulating a simple or compound amphiphilic matrix, possibly containing tensioactive substances and/or co-solvents.

The compounds of the invention are characterised by the presence of an accelerated phase of the quota of drug which under sink conditions continues to be rapid up to complete solubilisation, dispersion and/or emulsion of the system which rapidly makes the active principle available in the gastrointestinal tract.

Transportation with amphiphilic systems possibly formulated with tensioactive substances, co-solvents and other excipients, useful for imparting good technological properties to the pharmaceutical forms created in this way, makes it possible to increase the speed of dissolution in vitro and confers properties of potentially improved bioavailability and less variability in absorption.

The aim of the present invention is therefore to provide an oral formulation of a derivative of camptothecin which is not readily soluble in water.

As a derivative of camptothecin which is not readily soluble in water is meant any of the compounds reported in the section titled "Technical basis of the invention". Preferably this derivative is 7-[(E)-t-butyloxyminomethyl]-camptothecin (or gimatecan) in its amorphous form or in its crystalline forms I, II or III, as described before, and/or its pharmaceutically acceptable salts. Even more preferable is for the gimatecan to be its crystalline form I.

Examples of pharmaceutically acceptable salts are, in case of nitrogen atoms having basic character, the salts with pharmaceutically acceptable acids, both inorganic and organic, such as for example, hydrochloric acid, sulfuric acid, acetic acid, or, in the case of acid group, such as carboxyl, the salts with pharmaceutically acceptable bases, both inorganic and organic, such as for example, alkaline and alkaline-earth hydroxides, ammonium hydroxide, amine, also heterocyclic ones.

The invention provides rapid-release oral pharmaceutical compounds containing 7-[(E)-t-butyloxyminomethyl] camptothecin (gimatecan) as the active principle including a matrix consisting of liquid amphiphilic substances or with a melting point of less than 60°C in which the active principle is at least partially soluble and/or dispersed and/or inglobated.

According to a preferred embodiment of the invention, the compound of the invention also includes a tensioactive component which is compatible with the amphiphilic matrix capable of solubilising and/or dispersing homogeneously in the amphiphilic matrix.

According to an even more preferred embodiment, the compound of the invention also includes a component consisting of co-solvents capable of dispersing in the tensioactivated amphiphilic matrix or of being able in turn to be loaded by the amphiphilic matrix, either tensioactivated or not, to obtain a liquid, semisolid or solid form.

Any other excipients to improve the machinability of the pharmaceutical form may also be present.

By "amphiphilic substance" is meant a substance the molecules of which contain both a hydrophylic and a hydrophobic portion.

The amphiphilic substances which can be used according to the invention include polar lipids (lecithin, phosphatidylcholine, phosphatidylethanolamine) ceramides, glycol ialkyl ethers such as diethyleneglycol imonoethyl ethers (Transcutol^{®}), macrogol glycerides consisting of mixtures of mono-di and triglycerides and of mono and disters of polyethylene glycols and of fatty acids (Gelucire™ 44/14; Gelucire™ 50/14), hydroxystearate polyethylene glycols (Solutol^{®} HS 15), triglycerides of the C8-C10 fraction of coconut oil (Mygliol^{®} 810 N), polysorbates (Tween™ 20 - Tween™ 80), phosphatides (Phosal^{®}), hydrogenated castor oil POE 40 (Cremophor^{®} RH 40), monooleate esters of glycerol, linoleics (Peceol^{®}, Maisine^{®} 35-1), oily unsaturated polyglycosylated glycerides, capril-caproil (Labrafil^{®} M 1944, Labrasol^{®}), monolaurate polyethylene glycols (Lauroglycol^{®} FCC).

These substances may also be mixed with each other to obtain various melting or softening points alone or in the presence of an active principle.

Preferably the amphiphilic substance consists of macrogol glycerides, such as Gelucire™. It is even more preferable for the amphiphilic substance to be Gelucire™ 44/14, i.e. PEG-32 (polyethylene glycols with a mean molecular weight of between 1305 and 1595 Daltons) glyceryl laurate Gelucire™ 44/14 or Gelucire™ 50/13, i.e. PEG-32 (polyethylene glycols with a mean molecular weight of between 1305 and 1595 Daltons) glyceryl stearate).

The tensioactive substances which can be used according to the invention include the same phosphatides and lecithins (phosphatidylcholines, phosphatidyldiethanolamines, sfingomyelins), anionic and non-anionic emulsifying waxes, sodium lauryl sulphate, sodium dodecyl sulphate, polysorbates, cholic acids, poloxamers, sodium sulphosuccinate, sodium lauryl sarcosinate.

According to a general embodiment of the invention, first of all an amphiphilic matrix containing one or more amphiphilic materials to which one or more tensioactive substances are added to the soluble or molten mixture at temperatures in excess of 60°C is prepared. The quantity of tensioactive substance is usually not more than 10% w/w; preferably between 0,1% and 5%.

To this mixture it is possible to immediately add a variable quantity of co-solubilising substances such as water, polyethylene glycols, glycerine, up to 50% sorbitol; the optimum quantity is between 0.1% and 2.5 % to obtain a homogeneous dispersion.

The active principle can be solubilised and/or dispersed in this preparation up to a concentration of between 0.1 % and 50 %. The formulation obtained in this way could be used to fill hard or soft gelatin capsules.

According to a preferred embodiment of the invention, the said pharmaceutical compound is contained in hard gelatin capsules; such as the Licaps^{®}capsules, or soft gelatin capsules, softgel capsules.

The object of the present invention is also the method of preparation of the above-mentioned pharmaceutical compound and of the corresponding capsules.

The compounds of the invention can be obtained by a method consisting of he following stages:
a) First of all the semisolid amphiphilic excipients are possibly brought to melting point above 60°C; or one or more semisolid amphiphilic excipients are mixed bringing them to melting point until a solution and/or homogeneous dispersion is obtained which at ambient temperature turns semisolid or solid. To these excipients, which have been made liquid by melting or were already naturally liquid at ambient temperature, it is possible to add tensioactive excipients, in this or in other phases, until a homogeneous dispersion is obtained.
b) To the tensioactivated amphiphilic matrix obtained at point (a) the active principle is solubilised, dispersed and/or inglobated to obtain a homogeneous solution and/or dispersion.
c) To the system obtained at point (b) it is possible to add various quantities of co-solvents, such as water, polyethylene glycols, glycerin, sorbitol to obtain a homogeneous dispersion. The system obtained in this way can be loaded into hard or soft gelatin capsules so as to obtain a formulation which may be liquid, semisolid or solid inside the capsule.
d) To the systems thus obtained at point c), excipients with various functions can be added to convert any liquid or semisolid formulations into a completely solid phase for the preparation of capsules, tablets, granules, microgranules and sachets. These functional excipients may be silicics, celluloses, amides, sugars, polyvinylpyrrolidones, methacrylates and the more common smoothing agents, anti-clumping agents, lubricants such as magnesium stearate, stearic acid and talc.
e) Other adjuvants can be selected from preservatives (parabenes, benzalconium chloride) mineral and organic acids/bases, antioxidants ("butylated hydroxyanisole", BHA, and the related compound "butylated hydroxytoluene", BHT) or stabilisers ("ethylenediaminetetraacetic acid", EDTA).

An alternative way of preparing a pharmaceutical form may be to use the liquid or semisolid amphiphilic matrix as the granulating element. Once it has been brought to melting point this matrix contains the tensioactive substances, solubilised or dispersed, and the active principle for a percentage quota of the formulation. To these excipients may first have been added the remaining part of the active principle to obtain a solid compound ready to be divided into capsules, sachets or converted into tablets with the addition of suitable adjuvants such as silicics, microcrystalline celluloses, amides and lubricants. The semisolid amphiphilic matrix by cooling and with the aid of an extrusion and/or granulation process helps to compact the formulation until an easily workable or machinable granule or microgranule is obtained. A possible dry or wet granulation process can be used to produce the final pharmaceutical form.

The amphiphilic matrix possibly containing the tensioactive substances may contain all the pharmacologically active part of the active principle directly in solution and/or in suspension and/or in a dispersion.

Further excipients with various functions may be added to convert any liquid or semisolid formulations into the completely solid phase for the preparation of capsules, tablets, granules, microgranules and sachets. These functional excipients may be silicics, celluloses, amides, sugars, polyvinylpyrrolidones, methacrylates and the more common smoothing agents, anti-clumping agents, lubricants such as magnesium stearate, stearic acid and talc.

The compounds of the present invention may possibly include a gastro-soluble or gastro-resistant coating with derivatives of the celluloses and/or methacrylic acid polymers.

The capsules, microgranules and/or tablets can be subjected to well-known coating processes with gastro-soluble or gastro-protected films with celluloses and methacrylic acid polymers.

In terms of dissolution characteristics, when these formulations come into contact with water or aqueous fluids there is the immediate dispersion, solubilisation and/or emulsion of the system containing the principle formulated in this way. The tensioactive substances and the co-solvents present in the amphiphilic structure promote the wettability of the system and the passage into solution of the active principles leading to a potential increase in absorption in the gastrointestinal tract.

The following examples illustrate the invention in greater detail.

### EXAMPLES

### EXAMPLE 1

549.9 g of Gelucire™ 44/14 (PEG-32 glyceryl laurate (pale yellow)) was loaded into the melter and brought to melting point at a temperature of between 55°C and 65°C.

To the molten mass was added, under vigorous agitation, 0.1g of gimatecan until a homogeneous solution/dispersion was obtained.

The mixture obtained in this way was left under agitation at a temperature of at least 55°C for at least 15 minutes; then the O or double-O shaped hard gelatin capsules were filled using a distribution syringe, until a weight of 550 mg was reached per individual capsule.

Then the top of the capsule was placed on the body of the capsule to close it and it was sealed using a sealing system involving a 50% ethanol and water spray and then heated in hot air until the final capsules each containing a 0.1 mg dose were obtained.

The capsules obtained in this way exhibited a release in vitro of not less than 80% after 30 minutes according to the method described in USP/NF.

Using the same approach and reducing the quantity of Gelucire™ 44/14 proportionally capsules in the various dosages were obtained (0.1 mg - 0.25 mg - 0.5 mg).

For 1 mg capsules the quantity of Gelucire™ 44/14 was increased to 809 mg per capsule for a total weight of 810 mg.

| **Raw materials** | **0.1mg capsules** | **0.25mg capsules** | **0.5mg capsules** | **1mg capsules** |
|---|---|---|---|---|
| gimatecan | 0.1mg | 0.25mg | 0.5mg | 1 mg |
| Gelucire™ 44/14 | 549.9mg | 549.75 | 549.5mg | 809mg |
| Total | 550mg | 550g | 550mg | 810mg |

Other compounds replacing the Gelucire™ 44/14 with other amphiphilic vehicles were prepared subsequently keeping the quantity of excipients constant.

The various compounds are described below.

| **Raw materials INN/Comme rcial name** | **Chemical name/chemical composition** | **0.1 mg capsules** | **0.25mg capsules** | **0.5mg capsules** |
|---|---|---|---|---|
| gimatecan | 7-[(E)-t-butyloxyminomethyl] camptothecin | 0.1mg | 0.25mg | 0.5mg |
| Mygliol^{®} 810 N | Triglycerides of the C8-C10 fraction of coconut oil (colourless) | 549.9mg | 549.75 | 549.5mg |
| Transcutol^{®} | Diethylene glycol monoethyl ether (colourless) | 549.9mg | 549.75 | 549.5mg |
| Tween™ 80 | Polysorbate 80 (yellow) | 549.9mg | 549.75. | 549.5mg |
| Phosal^{®} | Phosphatides / proliposomes (pale yellow/visc.) | 549.9mg | 549.75 | 549.5mg |
| Cremophor^{®} RH40 | Hydrogenated castor oil POE 40 (white semisolid) | 549.9mg | 549.75 | 549.5mg |
| Peceol^{®} | Glycerol esters (Glycerol monooleate (yellow) | 549.9mg | 549.75 | 549.5mg |
| Maisine^{®} 35-1 | Glycerol esters (Linoleic glycerides) (colourless) | 549.9mg | 549.75 | 549.5mg |
| Labrafil^{®} M 1944 | Unsaturated polyglycosylated glycerides (oleolyl) (colourless) | 549,9mg | 549.75 | 549.5mg |
| Gelucire™ 50/13 | PEG-32 glyceryl stearate | 549.9mg | 549.75 | 549.5mg |
| Labrasol^{®} | Unsaturated polyglycosylated glycerides (capril-caproil) (pale yellow) | 549.9mg | 549.75 | 549.5mg |
| Lauroglycol^{®} FCC | Monolaurate polyethylene glycol (colourless) | 549.9mg | 549.75 | 549.5mg |
| Solutol^{®} H 15 | 660 12 - Hydroxystearate polyethylene glycol (whitish yellow paste) | 549.9mg | 549.75 | 549.5mg |
| Total | | 550mg | 550mg | 550mg |

### EXAMPLE 2

548.9 g of Gelucire™ 44/14 was loaded into the melter and brought to melting point at a temperature of between 55°C and 65°C.

To the molten mass were added, under vigorous agitation, first 1 g of BHT or BHA, then 0.1g of gimatecan until a homogeneous solution/dispersion was obtained.

The mixture obtained in this way was left under agitation, at a temperature of at least 55°C, for at least 15 minutes; then the O or double-O shaped hard gelatin capsules were filled using a distribution syringe, until a weight of 550 mg was reached per individual capsule.

Then the top of the capsule was placed on the body of the capsule to close it and it was sealed using a sealing system involving a 50% ethanol and water spray and then heated in hot air until the final capsules each containing a 0.1 mg dose were obtained.

The capsules obtained in this way exhibited a release in vitro of not less than 80% after 30 minutes according to the method described in USP/NF.

Using the same approach and reducing the quantity of Gelucire™ 44/14 proportionally capsules in the various dosages were obtained (0.1 mg - 0.25 mg - 0.5 mg ).

For 1 mg capsules the quantity of Gelucire™ 44/14 was increased to 809 mg per capsule for a total weight of 810 mg.

| **Raw materials** | **0.1mg capsules** | **0.25mg capsules** | **0.5mg capsules** | **1mg capsules** |
|---|---|---|---|---|
| gimatecan | 0.1mg | 0.25mq | 0.5mg | 1mg |
| Gelucire™ 44/14 | 548.9mg | 548.75 | 548.5mg | 808mg |
| BHT/BHA | 1mg | 1mg | 1mg | 1mg |
| Total | 550mg | 550mg | 550mg | 810mg |

### EXAMPLE 3

499.9 g of Gelucire™ 50/13 was loaded into the melter and brought to melting point at a temperature of between 55°C and 65°C.

To the molten mass was added, under vigorous agitation, 0.1g of gimatecan until a homogeneous solution/dispersion was obtained.

To the mixture obtained, still under vigorous agitation, was added 5 g of sodium lauryl sulphate and 45 g of polyethylene glycol 1000 previously brought to melting point.

The mixture obtained in this way was left under agitation, at a temperature of at least 55°C, for at least 15 minutes; then the O or double-O shaped hard gelatin capsules were filled using a distribution syringe, until a weight of 600 mg was reached per individual capsule.

Then the top of the capsule was placed on the body of the capsule to close it and it was sealed using a sealing system involving a 50% ethanol and water spray and then heated in hot air until the final capsules were obtained.

The capsules obtained in this way exhibited a release in vitro of not less than 80% after 30 minutes according to the method described in USP/NF.

Using the same approach and reducing the quantity of Gelucire™ 50/13 proportionally capsules in the various dosages were obtained (0.1 mg - 0.25 mg - 0.5 mg).

For 1 mg capsules the quantity of Gelucire™ 44/14 was increased to 809 mg per capsule for a total weight of 810 mg.

| **Raw materials** | **0.1 mg capsules** | **0.25mg capsules** | **0.5mg capsules** | **1mg capsules** |
|---|---|---|---|---|
| gimatecan | 0.1 mg | 0.25mg | 0.5mg | 1mg |
| Gelucire™ 50/13 | 549.9mg | 549.75 | 549.5mg | 759mg |
| Sodium lauryl sulphate | 5mg | 5mg | 5mg | 5mg |
| PEG 1000 | 45mg | 45mg | 450mg | 45mg |
| Total | 600mg | 600mg | 600mg | 810mg |

### EXAMPLE 4

500 g of Gelucire™ 44/14 and 39 g of Solutol^{®} HS 15 was loaded into the melter and brought to melting point at a temperature of between 55°C and 65°C.

To the molten mass was added, under vigorous agitation, 1 g of gimatecan until a homogeneous solution/dispersion was obtained.

To the mixture obtained, still under vigorous agitation, were added 5 g of sodium lauryl sulphosuccinate and 5 g of polyethylene glycol 1000.

The mixture obtained in this way was left under agitation, at a temperature of at least 55°C, for at least 15 minutes; then the O or double-O shaped hard gelatin capsules were filled using a distribution syringe, until a weight of 550 mg was reached per individual capsule.

Then the top of the capsule was placed on the body of the capsule to close it and it was sealed using a sealing system involving a 50% ethanol and water spray and then heated in hot air until the final capsules were obtained.

The capsules obtained in this way exhibited a release in vitro of not less than 75% after 45 minutes according to the method described in USP/NF.

### EXAMPLE 5

509.9 g of Gelucire™ 44/14 was loaded into the melter and brought to melting point at a temperature of between 55°C and 65°C, to which was added 5 g of diethylene glycol monoethylether (Transcutol^{®}).

To the molten mass was added, under vigorous agitation, 0.1 g of gimatecan until a homogeneous solution/dispersion was obtained.

To the mixture obtained, still under vigorous agitation, were added 5 g of Peceol^{®} and 30 g of Labrasol^{®}.

The mixture obtained in this way was left under agitation, at a temperature of at least 55°C, for at least 15 minutes; then the O or double-O shaped hard gelatin capsules were filled using a distribution syringe, until a weight of 580 mg was reached per individual capsule.

Then the top of the capsule was placed on the body of the capsule to close it and it was sealed using a sealing system involving a 50% ethanol and water spray and then heated in hot air until the final capsules were obtained.

The capsules obtained in this way exhibited a release in vitro of not less than 75% after 45 minutes in a dissolution bath containing 900 ml of 0.1 N hydrochloric acid with a paddle rotating at 50 rpm.

### EXAMPLE 6

100 g of Gelucire™ 44/14 was loaded into a mixer/melter and brought to melting point at a temperature of between 55°C and 65°C, together with 5 g of Solutol^{®} HS15.

To the molten mass was added, under vigorous agitation, 0.5 g of gimatecan until a homogeneous solution/dispersion was obtained.

To the mixture obtained, still under vigorous agitation, was added 4 g of sodium dodecyl sulphate.

499 g of microcrystalline cellulose together with a further 0.5 g of gimatecan were loaded in a granulator/homogeniser. Appropriate mixing was carried out for at least 15 minutes.

The molten mass prepared earlier was added to the granulator containing the microcrystalline cellulose and the gimatecan and the whole was mixed until homogenous granules were formed.

The granules obtained were unloaded and after normalisation were loaded into the mixer to which was added around 100 g of microcrystalline cellulose, 0.5 g of magnesium stearate and 0.5 g of colloidal silica.

After having mixed the mixture for 5 minutes, the final mixture was tabletted at the final weight of 710 mg/ tablet. The tablets obtained in this way, subjected to dissolution tests, in a simulated gastric environment, exhibited a release of the active principle of not less than 75% after 45 minutes.

### EXAMPLE 7

50 g di Gelucire™ 50/14 was loaded into a mixer/melter and brought to melting point at a temperature of between 60°C and 65°C.

To the molten mass was added, under vigorous agitation, 0.5 g of gimatecan until a homogeneous solution/dispersion was obtained.

To the mixture obtained, still under vigorous agitation, was added 4 g of soya lecithin.

405 g of lactose monohydrate together with a further 0.5 g of gimatecan were loaded in a granulator/homogeniser. Appropriate mixing was carried out for at least 15 minutes.

The molten mass prepared earlier was added to the granulator containing lactose and gimatecan and the whole was mixed until homogenous granules were formed.

The granules obtained were unloaded and after normalisation were loaded into a mixer to which were added around 174 g of microcrystalline cellulose, 1 g of magnesium stearate and 25 g of colloidal silica.

After having mixed the mixture for 5 minutes, the final mixture was tabletted at the final weight of 660 mg/ tablet. The tablets obtained in this way, subjected to dissolution tests, in a simulated gastric environment, exhibited a release of the active principle of not less than 80% after 45 minutes.

## Claims

1. Immediate-release pharmaceutical formulation for oral use containing 7-[(E)-t-butyloxyiminomethyl] camptothecin (gimatecan) as the active principle and including a matrix consisting of liquid amphiphilic substances with a melting point of less than 60°C, in which the active principle is at least partially dissolved and/or dispersed and/or inglobated.

2. The pharmaceutical formulation according to claim 1, in which the gimatecan is in crystalline form I.

3. The pharmaceutical formulation according to claims 1 or 2, further including a tensioactive component compatible with the soluble amphiphilic matrix and/or dispersible homogeneously in the amphiphilic matrix.

4. The pharmaceutical formulation according to any one of the preceding claims, also including co-solvents dispersible in the tensioactivated amphiphilic matrix.

5. The pharmaceutical formulation according to any one of the preceding claims, in which the amphiphilic matrix is selected from the group containing: polar lipids, ceramides, glycolyalkyl ethers, macrogol glycerides, hydroxystearate polyethylene glycols, triglycerides of the C₈-C₁₀ fraction of coconut oil, polysorbates, phosphatides, hydrogenated castor oil, esters of monooleate glycerol, linoleics, oily unsaturated polyglycosylated glycerides, capril-caproil, monolaurate polyethylene glycols and their mixtures.

6. The pharmaceutical formulation according to any one of the preceding claims, in which the amphiphilic matrix is a Gelucire™.

7. The pharmaceutical formulation according to any one of the preceding claims, in which the tensioactive component is chosen from the group containing: phosphatides and lecithins, anionic and non-ionic emulsifying waxes, sodium lauryl sulphate, sodium dodecyl sulphate, polysorbates, cholic acids, poloxamers, sodium sulphosuccinate and sodium lauryl sarcosinate; and is present in a quantity of not more than 10% in weight.

8. The pharmaceutical formulation according to any one of the preceding claims, in which the active principle is present in a quantity of between 0.1 % and 50%.

9. The pharmaceutical formulation according to any one of the preceding claims in liquid, semisolid or solid form.

10. A capsule containing the pharmaceutical formulation according to any one of the preceding claims.

11. The capsule according to claim 10 in soft or hard gelatin.

12. Process for the preparation of the formulations of claims 1 to 9 which includes the total or partial solubilisation, suspension, dispersion or inglobation of the active principle with the amphiphilic matrix at temperatures in excess of 60°C.

13. Process for the preparation of the capsule according to claims 10 or 11, which includes the addition of the pharmaceutical formulation of claims 1 to 9 to one of the two cavities of the open capsule and sealing of the capsule.

## Patentansprüche

1. Pharmazeutische Zubereitung mit sofortger Freisetzung zur oralen Verwendung, welche 7-[(E)-t-Butyloxyiminomethyl]camptothecin (Gimatecan) als Wirkstoff enthält, und welche eine Matrix einschließt, die aus flüssigen amphiphilen Substanzen mit einem Schmelzpunkt von weniger als 60 °C besteht, in welcher der Wirkstoff wenigstens teilweise gelöst und/oder dispergiert und/oder inglobuliert ist.

2. Pharmazeutische Zubereitung, gemäß Anspruch 1, in welcher das Gimatecan in der kristallinen Form I vorliegt.

3. Pharmazeutische Zubereitung, gemäß den Ansprüchen 1 oder 2, welche weiterhin eine oberflächenaktive Komponente einschließt, die mit der löslichen amphiphilen Matrix kompatibel ist und/oder homogen in der amphiphilen Matrix dispergierbar ist.

4. Pharmazeutische Zubereitung, gemäß einem der vorangehenden Ansprüche, welche auch Co-Lösemittel einschließt, die in der oberflächenaktiven amphiphilen Matrix dispergierbar sind.

5. Pharmazeutische Zubereitung, gemäß einem der vorangehenden Ansprüche, in welcher die amphiphile Matrix ausgewählt ist aus der Gruppe, enthaltend: polare Lipide, Ceramide, Glkyolalkylether, Macrogolglyceride, Hydroxystearatpolyethylenglykole, Triglyceride der C₈-C₁₀-Fraktion von Kokosnussöl, Polysorbate, Phosphatide, hydriertes Rizinusöl, Ester von Glycerinmonooleat, Linolsäuren, ölhaltige ungesättigte polyglycosylierte Glyceride, Capril-Caproil, Monolauratpolyethylenglykole und deren Mischungen.

6. Pharmazeutische Zubereitung, gemäß einem der vorangehenden Ansprüche, in welcher die amphiphile Matrix eine Gelucire^{™}-Matrix ist.

7. Pharmazeutische Zubereitung, gemäß einem der vorangehenden Ansprüche, in welcher die oberflächenaktive Komponente ausgewählt ist aus der Gruppe, enthaltend: Phosphatide und Lecithine, anionische und nichtionische emullgierende Wachse, Natriumlaurylsulfat, Natriumdodecylsulfat, Polysorbate, Cholsäuren, Poloxamere, Natriumsulfosuccinat und Natriumlauroylsarcosinat, und in einer Menge von nicht mehr als 10 Gewichts-% vorhanden ist.

8. Pharmazeutische Zubereitung, gemäß einem der vorangehenden Ansprüche, in welcher der Wirkstoff in einer Menge von zwischen 0,1 % und 50 % vorliegt.

9. Pharmazeutische Zubereitung, gemäß einem der vorangehenden Ansprüche, in flüssiger, halbfester oder fester Form.

10. Kapsel, enthaltend die pharmazeutische Zubereitung gemäß einem der vorangehenden Ansprüche.

11. Kapsel, gemäß Anspruch 10, aus weicher oder harter Gelatine.

12. Verfahren zur Herstellung der Zubereitungen der Ansprüche 1 bis 9, welches die vollständige oder teileweise Solubilisierung, Suspension, Dispersion oder Inglobulierung des Wirkstoffs mit der amphiphilen Matrix bei Temperaturen über 60 °C einschließt.

13. Verfahren zur Herstellung der Kapsel, gemäß den Ansprüchen 10 oder 11, welches das Hinzufügen der pharmazeutischen Zubereitung der Ansprüche 1 bis 9 in einen der zwei Hohlräume der offenen Kapsel und das Versiegeln der Kapsel umfasst.

## Revendications

1. Formulation pharmaceutique à libération immédiate pour usage oral contenant de la 7-[(E)-t-butyloxyiminométhyl] camptothécine (gimatecan) en tant que principe actif et comprenant une matrice constituée de substances amphiphiles liquides présentant un point de fusion inférieur à 60°C, dans laquelle le principe actif est au moins en partie dissous et/ou dispersé et/ou englobé.

2. Formulation pharmaceutique selon la revendication 1, dans laquelle le gimatecan est sous la forme cristalline I.

3. Formulation pharmaceutique selon la revendication 1 ou la revendication 2, comprenant en outre un composant tensioactif compatible avec la matrice amphiphile soluble et/ou dispersible de manière homogène dans la matrice amphiphile.

4. Formulation pharmaceutique selon l'une quelconque des revendications précédentes, comprenant également des co-solvants dispersibles dans la matrice amphiphile tensioactivée.

5. Formulation pharmaceutique selon l'une quelconque des revendications précédentes, dans laquelle la matrice amphiphile est choisie dans le groupe contenant : des lipides polaires, des céramides, des glycoalkyléthers, des macrogol glycérides, des hydroxystéarates de polyéthylèneglycols, des triglycérides de la fraction en C₈-C₁₀ d'huile de noix de coco, des polysorbates, des phosphatides, de l'huile de ricin hydrogénée, des esters de monooléate de glycérol, linoléiques, des glycérides polyglycosylés d'huiles insaturées, groupe capryle-caproyle, des monolaurates de polyéthylèneglycols et des mélanges de ceux-ci.

6. Formulation pharmaceutique selon l'une quelconque des revendications précédentes, dans laquelle la matrice amphiphile est un Gelucire^{™}.

7. Formulation pharmaceutique selon l'une quelconque des revendications précédentes dans laquelle le composant tensioactif est choisi dans le groupe contenant : des phosphatides et des lécithines, des cires anioniques et non ioniques émulsifiantes, du laurylsulfate de sodium, du dodécylsulfate de sodium, des polysorbates, des acides choliques, des Poloxamer, du sulfosuccinate de sodium et du laurylsarcosinate de sodium ; et est présent en une quantité n'excédant pas 10 % en poids.

8. Formulation pharmaceutique selon l'une quelconque des revendications précédentes, dans laquelle le principe actif est présent en une quantité entre 0,1 % et 50 %.

9. Formulation pharmaceutique selon l'une quelconque des revendications précédentes sous une forme liquide, semi-solide ou solide.

10. Gélule contenant la formulation pharmaceutique selon l'une quelconque des revendications précédentes.

11. Gélule selon la revendication 10 en gélatine molle ou dure.

12. Procédé de préparation des formulations des revendications 1 à 9 qui comprend la solubilisation, suspension, dispersion ou inclusion totale ou partielle du principe actif dans la matrice amphiphile à des températures supérieures à 60°C.

13. Procédé de préparation de la gélule selon les revendications 10 ou 11, qui comprend l'introduction de la formulation pharmaceutique des revendications 1 à 9 dans l'une des deux cavités de la gélule ouverte et le scellement de la gélule.
